(19) Europäisches Patentamt
European Patent Office
Office européen des brevets

(11) Publication number: **0 273 417 B1**

(12) # EUROPEAN PATENT SPECIFICATION

(45) Date of publication of patent specification: **21.08.91**

(51) Int. Cl.5: **C07D 513/04**, A01N 43/90, //(C07D513/04,285:00,237:00)

(21) Application number: **87119233.2**

(22) Date of filing: **24.12.87**

(54) **Thiadiazabicyclononane derivatives and herbicidal compositions.**

(30) Priority: **24.12.86 JP 310423/86**
**26.09.87 JP 242300/87**
**29.12.86 JP 312933/86**
**30.09.87 JP 246827/87**

(43) Date of publication of application:
**06.07.88 Bulletin 88/27**

(45) Publication of the grant of the patent:
**21.08.91 Bulletin 91/34**

(84) Designated Contracting States:
**BE CH DE ES FR GB IT LI NL**

(56) References cited:

**CHEMICAL ABSTRACTS, vol. 107, no. 15, 12th October 1987, page 725, abstract no. 134316u, Columbus, Ohio, US; & JP - A- 62 00091 (Cat. X,P)**

**JOURNAL OF ORGANIC CHEMISTRY, vol. 39, no. 20, 1974, pages 2951-2956; S.W. MOJE et al.: "Syntheses and reactions of 3,4-dialkyl-1,3,4-thiadiazolidine-2,5-diones"**

(73) Proprietor: **KUMIAI CHEMICAL INDUSTRY CO., LTD.**
**4-26, Ikenohata 1-chome**
**Taitoh-ku Tokyo 110(JP)**

Proprietor: **IHARA CHEMICAL INDUSTRY CO., LTD.**
**4-26, Ikenohata 1-chome**
**Taitoh-ku, Tokyo(JP)**

(72) Inventor: **Yamaguchi, Mikio**
**132, Kojima Fukude-cho**
**Iwata-gun Shizuoka-ken(JP)**
Inventor: **Watase, Yukihiro**
**531-14, Nakahara**
**Shizuoka-shi Shizuoka-ken(JP)**
Inventor: **Kambe, Takeshi**
**C-201, 46-20, Ohara-cho**
**Itabashi-ku Tokyo(JP)**
Inventor: **Katou, Susumu**
**11-23, Mabuchi 4-chome**
**Shizuoka-shi Shizuoka-ken(JP)**

(74) Representative: **Wächtershäuser, Günter, Dr.**
**Tal 29**
**W-8000 München 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 273 417 B1**

## Description

The present invention relates to 9-phenylimino-8-thia-1,6-diazabicyclo-[4.3.0]nonane-7-one derivatives useful as herbicides and herbicidal compositions containing them.

In recent years, a number of herbicides have been developed and actually used, and they have contributed to the reduction of the agricultural work load and to the improvement of the productivity. As a herbicide having a hetero ring, oxadiazon [i.e. 5-t-butyl-3-(2,4-dichloro-5-isopropoxyphenyl)-1,3,4-oxadiazoline-2-one] is widely used. However, oxadiazon is likely to bring about phytotoxicity, when used in paddy fields, and it is not effective against perennial weeds, particularly against Sagittaria pygmaea. When used in upland fields, it is not so safe against crop plants such as corn or soybean and has drawbacks that its herbicidal activities are low against hardly controllable seeds such as cocklebur or morning-glory and against pigweed and lambsquaters. Accordingly, a development of a herbicide having improved herbicidal activities and safety has been desired.

Under the circumstances, the present inventors have conducted extensive researches with an aim to develop a herbicide which satisfies the following conditions, and have finally accomplished the present invention.

(1) It is effective at a low dose.
(2) It is effective against paddy field weeds and (or) against upland field weeds.
(3) It is also effective against perennial weeds and (or) hardly controllable weeds.
(4) It is effective in a wide range covering the germination stage to the growing stage.
(5) It has excellent residual effects and can be expected to provide stabilized effects.
(6) It is highly safe to crop plants.

Thus, the present invention provides a 9-phenylimino-8-thia-1,6-diazabicyclo [4.3.0]nonane-7-one derivative having the formula:

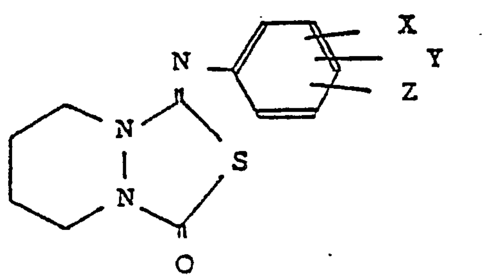

(I)

wherein each of X and Y is hydrogen or halogen such as chlorine, bromine, fluorine or iodine, and Z is

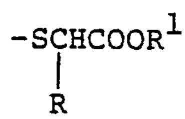

wherein R is hydrogen or alkyl, preferably $C_1$-$C_6$ alkyl, $R^1$ is alkyl, preferably $C_1$-$C_6$ alkyl, cycloalkyl, preferably $C_5$-$C_6$ cycloalkyl, or alkoxyalkyl, preferably $C_1$-$C_4$ alkoxy-$C_1$-$C_4$ alkyl, or -COOQ wherein Q is alkyl, preferably $C_1$-$C_6$ alkyl, or Y and Z together form

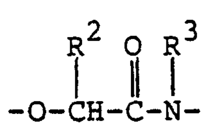

bonded to the phenyl ring, wherein $R^2$ is hydrogen or alkyl, preferably $C_1$-$C_6$ alkyl, and $R^3$ is alkyl, preferably $C_1$-$C_6$ alkyl, alkenyl, preferably $C_3$-$C_5$ alkenyl, or alkynyl, preferably $C_3$-$C_5$ alkynyl.

Further, the present invention provides a herbicidal composition comprising a herbicidally effective amount of a compound of the formula I as defined above and a carrier.

Now, the present invention will be described in detail with reference to the preferred embodiments.

In the accompanying drawing, Figure 1 is the infrared spectrum of Compound No. 2.

In the formula I, Z is preferably

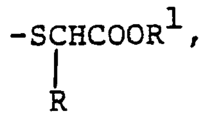

more preferably $-SCH_2COOR^1$ or

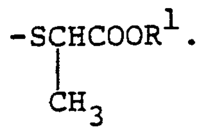

$R^1$ is preferably alkyl or cycloalkyl. It is also preferred that X is hydrogen or halogen and Y is halogen.

A compound having the formula:

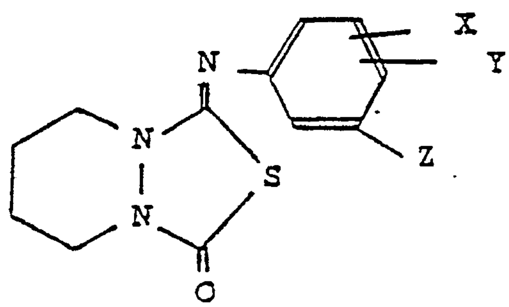

wherein X, Y and Z are as defined above, is particularly effective as a herbicide. Particularly preferred in this respect is a compound of the formula:

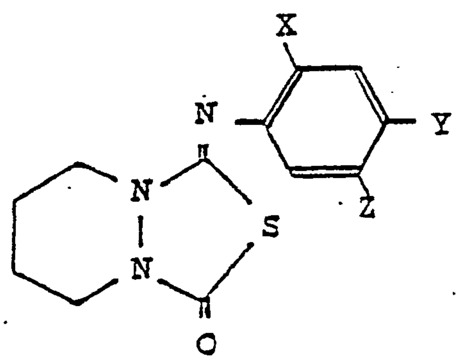

wherein X, Y and Z are as defined above and especially wherein X is hydrogen or halogen, particularly fluorine, Y is halogen, particularly chlorine, and Z is as defined above, particularly $-SCH_2COOR^1$ or

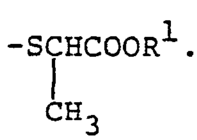

Typical Examples of the formula I will be presented in Table 1. The compound Nos. used in Table 1 will be referred to in the sebsequent description.

Table 1

| Compound No. | 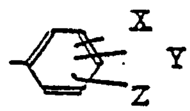 | Physical property m.p. (°C) or refractive index ($n_D^{20}$) |
|---|---|---|
| 1 | 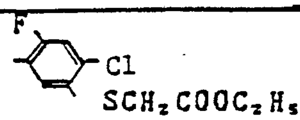 | 1.5990 |
| 2 | 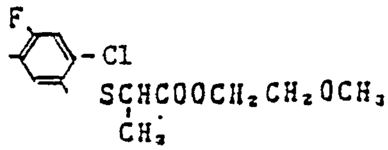 | 1.5814 |
| 3 | 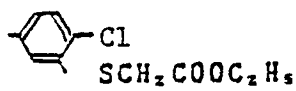 | 1.6201 |
| 4 | 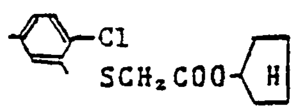 | 1.6105 |

Table 1 (continued)

| Compound No. | 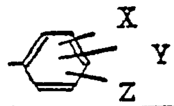 | Physical property m.p. (°C) or refractive index ($n_D^{20}$) |
|---|---|---|
| 5 | 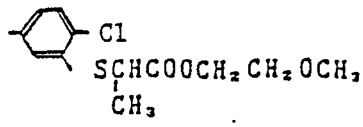 | 1.5960 |
| 6 | 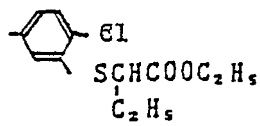 | 1.6030 |
| 7 | 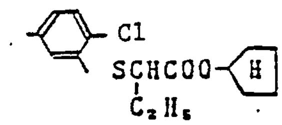 | 1.5975 |
| 8 | 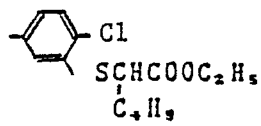 | 1.5790 |
| 9 | 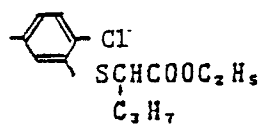 | 1.5990 |
| 10 | 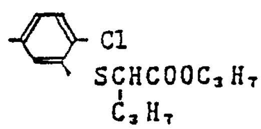 | 1.5914 |
| 11 | 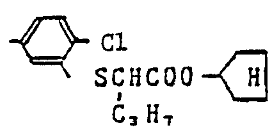 | 1.5872 |
| 12 | 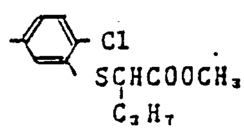 | 1.5815 |
| 13 | 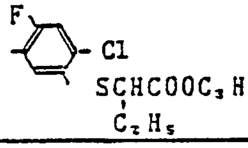 | 1.5886 |

Table 1 (continued)

| Compound No. | 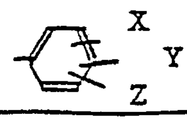 | Physical property m.p. (°C) or refractive index ($n_D^{20}$) |
|---|---|---|
| 14 | 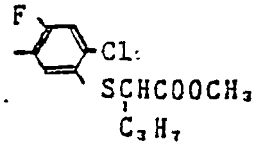 | 1.5749 |
| 15 | 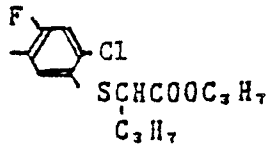 | 1.5708 |
| 16 | 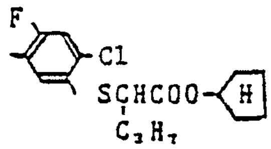 | 1.5840 |
| 17 | 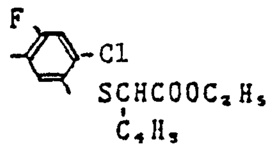 | 1.5680 |
| 18 | 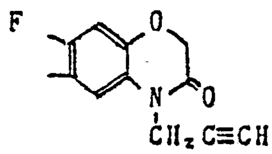 | 162—165 |
| 19 | 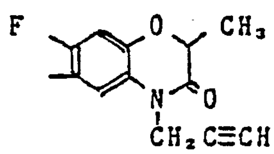 | 157—159 |
| 20 | 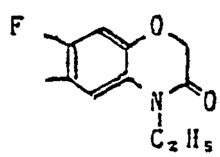 | 109—111 |
| 21 | 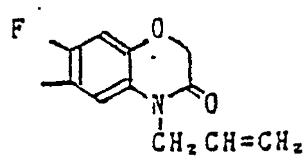 | 140—142 |

Table 1 (continued)

| Compound No. | 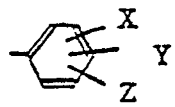 | Physical property m.p. (°C) or refractive index ($n_D^{20}$) |
|---|---|---|
| 22 | 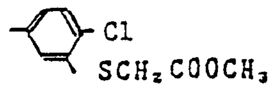 | 1.6250 |
| 23 | 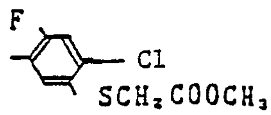 | 99 – 101 |
| 24 | 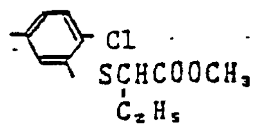 | 1.5980 |
| 25 | 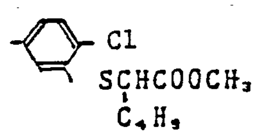 | 1.5920 |
| 26 | 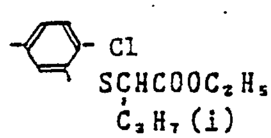 | 1.5909 |
| 27 | 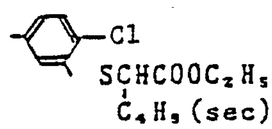 | 1.5880 |
| 28 | 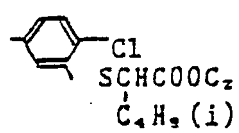 | 1.5720 |
| 29 | 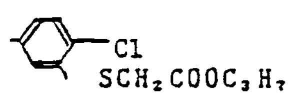 | 83 – 84 |
| 30 | 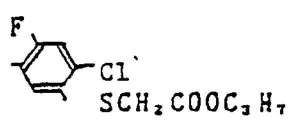 | 1.6040 |

Table 1 (continued)

| Compound No. | 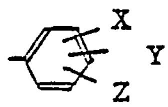 | Physical property m.p. (°C) or refractive index ($n_D^{20}$) |
|---|---|---|
| 31 | 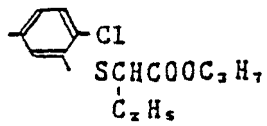 | 1.6011 |
| 32 | 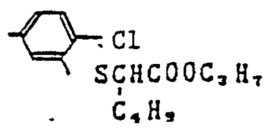 | 1.5815 |
| 33 | 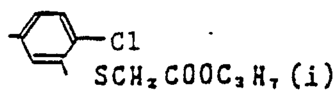 | 88–89 |
| 34 | 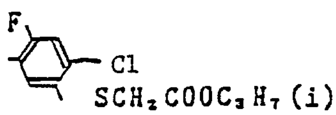 | 1.6039 |
| 35 | 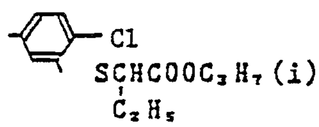 | 1.5828 |
| 36 | 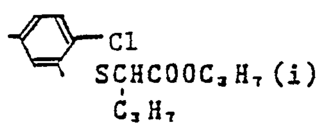 | 1.5871 |
| 37 | 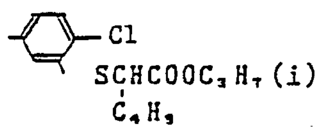 | 1.5810 |
| 38 | 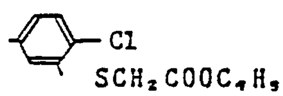 | 58–60 |
| 39 | 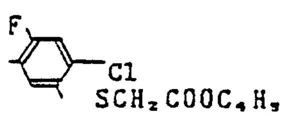 | 1.5985 |

Table 1 (continued)

| Compound No. | 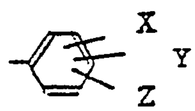 | Physical property m.p. (°C) or refractive index ($n_D^{20}$) |
|---|---|---|
| 40 | 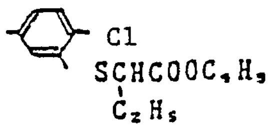 | 1.5750 |
| 41 | 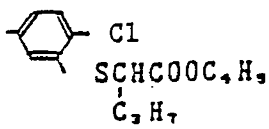 | 1.5837 |
| 42 | 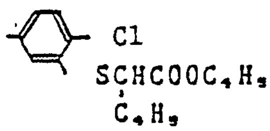 | 1.5810 |
| 43 | 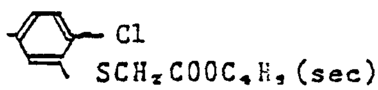 | 83−84 |
| 44 | 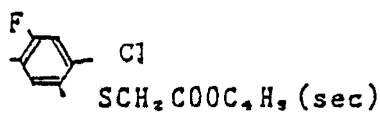 | 1.6240 |
| 45 | 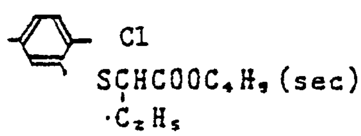 | 1.5835 |
| 46 | 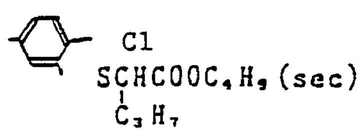 | 1.5807 |
| 47 | 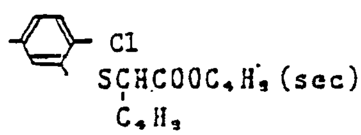 | 1.5710 |
| 48 | 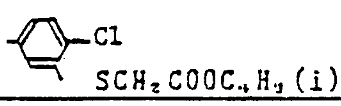 | 77−78 |

Fig. 9(B)

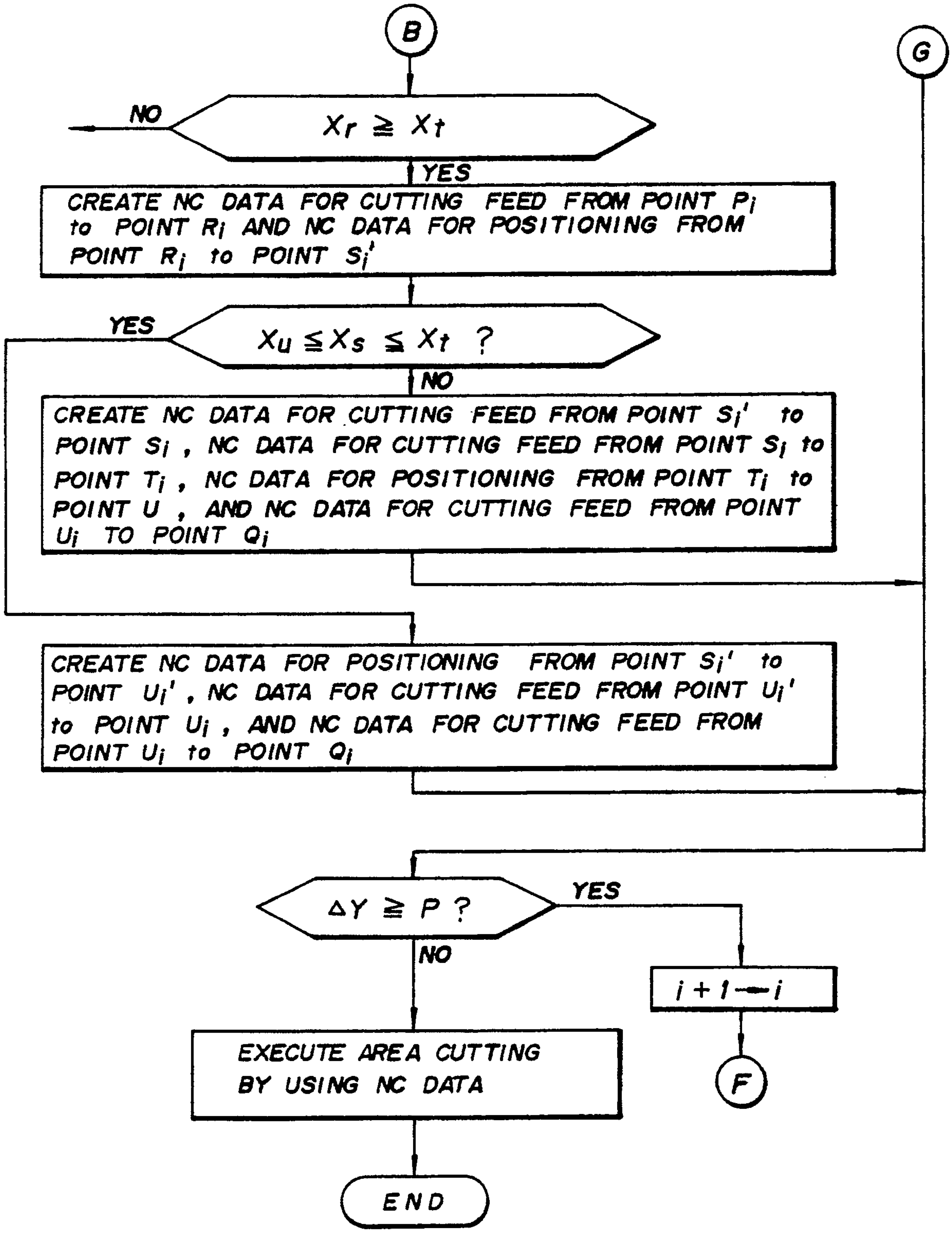
B
G
NO
Xr ≧ Xt
YES
CREATE NC DATA FOR CUTTING FEED FROM POINT Pi to POINT Ri AND NC DATA FOR POSITIONING FROM POINT Ri to POINT Si'
YES
Xu ≦ Xs ≦ Xt ?
NO
CREATE NC DATA FOR CUTTING FEED FROM POINT Si' to POINT Si , NC DATA FOR CUTTING FEED FROM POINT Si to POINT Ti , NC DATA FOR POSITIONING FROM POINT Ti to POINT U , AND NC DATA FOR CUTTING FEED FROM POINT Ui TO POINT Qi
CREATE NC DATA FOR POSITIONING FROM POINT Si' to POINT Ui' , NC DATA FOR CUTTING FEED FROM POINT Ui' to POINT Ui , AND NC DATA FOR CUTTING FEED FROM POINT Ui to POINT Qi
ΔY ≧ P ?
YES
NO
i + 1 → i
F
EXECUTE AREA CUTTING BY USING NC DATA
END

Table 1 (continued)

| Compound No. | 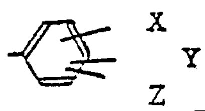 | Physical property m.p. (°C) or refractive index ($n_D^{20}$) |
|---|---|---|
| 58 | 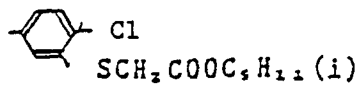 | 1.5965 |
| 59 | 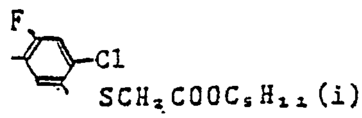 | 1.5870 |
| 60 | 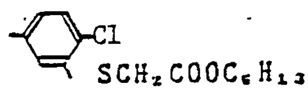 | 1.5766 |
| 61 | 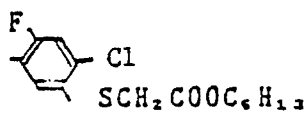 | 1.5759 |
| 62 | 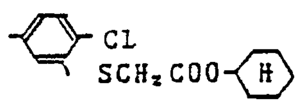 | 82—83 |
| 63 | 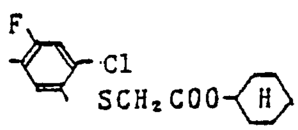 | 1.6015 |
| 64 | 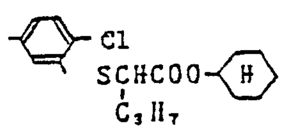 | 1.5872 |
| 65 | 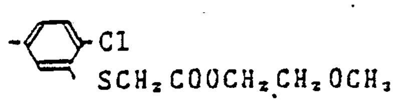 | 91—92 |
| 66 | 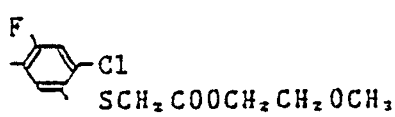 | 1.6041 |

Table 1 (continued)

| Compound No. | 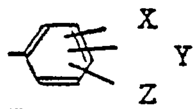 | Physical property m.p. (°C) or refractive index ($n_D^{20}$) |
|---|---|---|
| 67 | 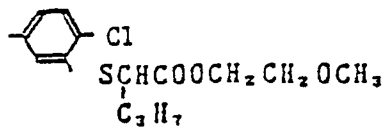 | 1.5904 |
| 68 | 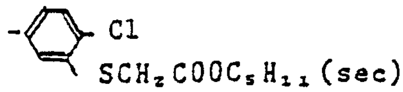 | 1.5928 |
| 69 | 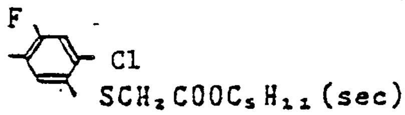 | 1.5844 |
| 70 | 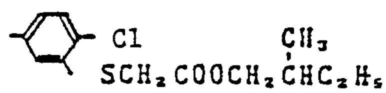 | 1.5935 |
| 71 | 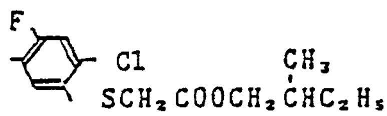 | 1.5865 |
| 72 | 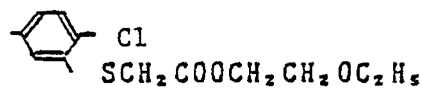 | 54—56 |
| 73 | 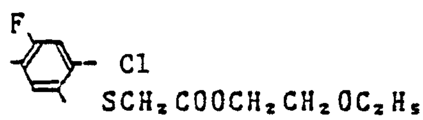 | 1.5962 |
| 74 | 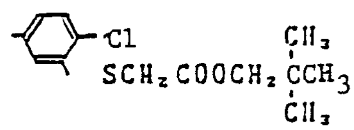 | 1.5941 |
| 75 | 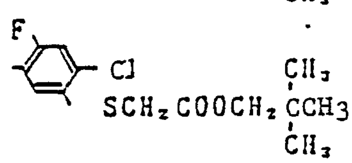 | 1.5845 |

Table 1 (continu...)

| Compound No. | 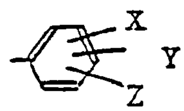 | Physical property m.p. ($^{o}C$) or refractive index ($n_D^{20}$) |
|---|---|---|
| 76 | 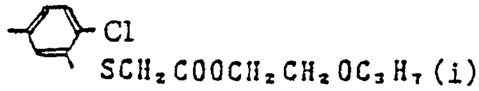 | 1.6000 |
| 77 | 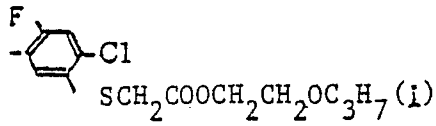 | 61–63 |
| 78 | 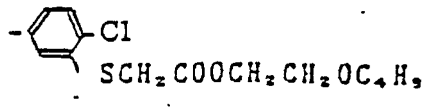 | 1.5820 |
| 79 | 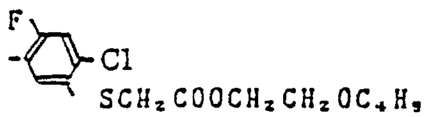 | 1.5882 |

Table 1 (continued)

| Compound No. | 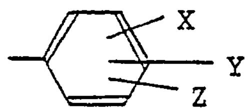 | Physical property m.p. (°C) or refractive index ($n_D^{20}$) |
|---|---|---|
| 80 | 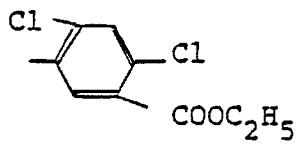 | 81-83 |
| 81 | 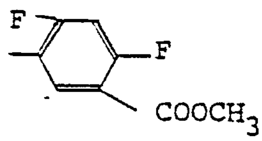 | 112-114 |
| 82 | 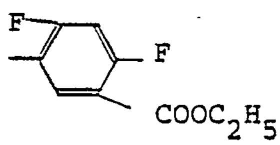 | 91-92 |
| 83 | 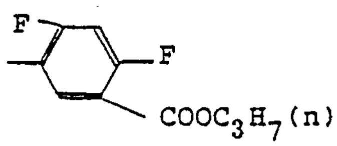 | 67-69 |
| 84 | 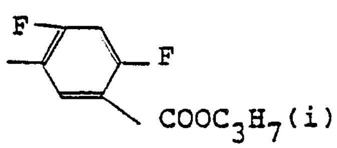 | 86-88 |
| 85 | 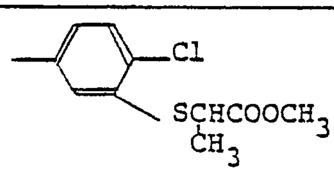 | 1.6035 |
| 86 | 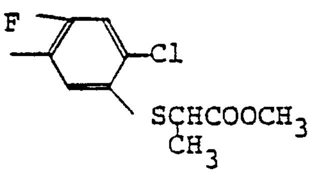 | 1.6055 |

Table 1 (continuted)

| Compound No. | 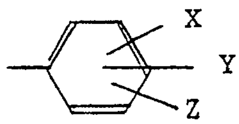 | Physical property m.p. ($^{o}C$) or refrective index ($n_D^{20}$) |
|---|---|---|
| 87 | 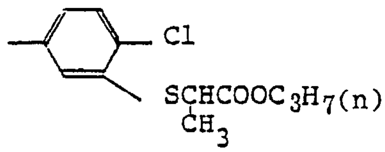 | 1.5955 |
| 88 | 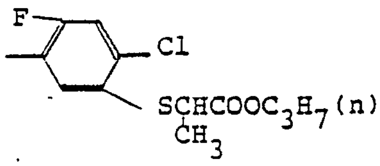 | 1.5929 |
| 89 | 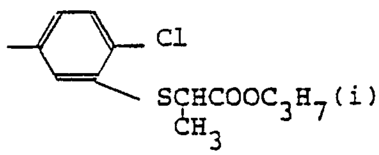 | 1.5918 |
| 90 | 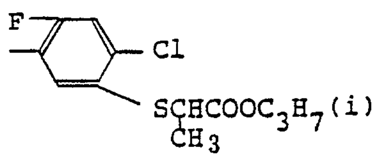 | 1.5820 |
| 91 | 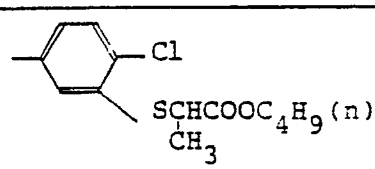 | 1.5922 |
| 92 | 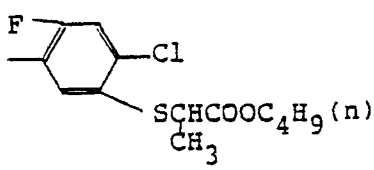 | 1.5749 |
| 93 | 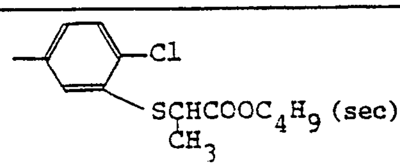 | 1.5861 |

Table 1 (continuted)

| Compound No. | 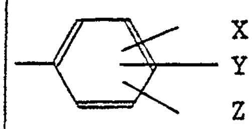 | Physical property m.p. (°C) or refrective index ($n_D^{20}$) |
|---|---|---|
| 94 | 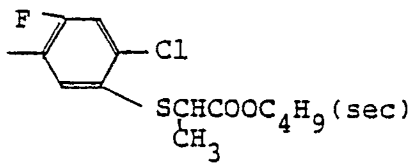 | 1.5755 |
| 95 | 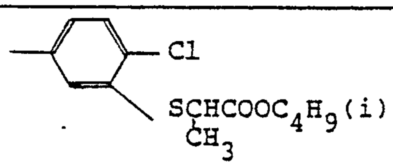 | 1.5860 |
| 96 | 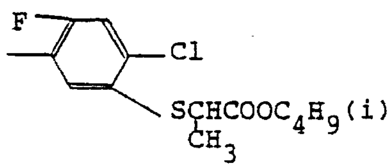 | 1.5700 |
| 97 | 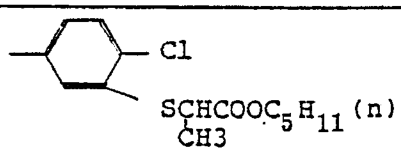 | 1.5820 |
| 98 | 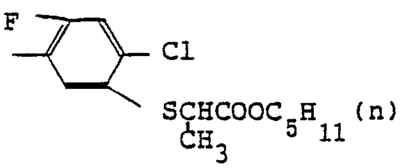 | 1.5760 |
| 99 | 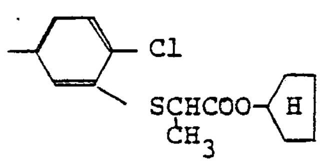 | 1.6071 |
| 100 | 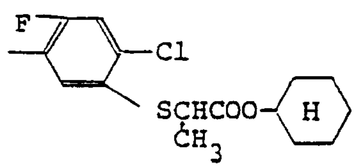 | 1.5780 |

The compound of the formula I of the present invention can be prepared by the following process.

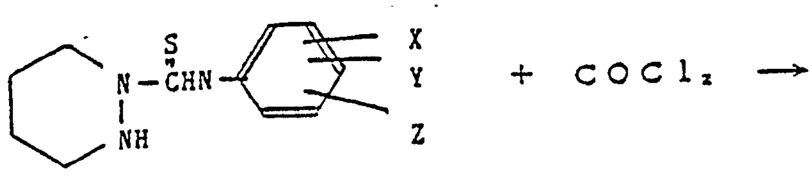


(II) (III)

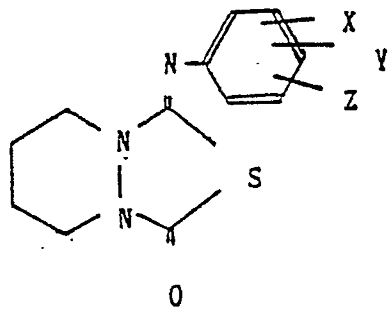

(I)

In the above formulas, X, Y and Z are as defined above. This process can be conducted by reacting the compound of the formula II with the compound of the formula III in the presence or absence of a base.

As the base, there may be mentioned an aliphatic tertiary amine such as triethylamine or trimethylamine; an aromatic tertiary amine such as pyridine, picoline or quinoline; or an inorganic base such as sodium hydroxide, potassium hydroxide, potassium carbonate or sodium carbonate.

The above reaction is preferably conducted in a solvent. As such a solvent, there may be mentioned a chlorine-containing hydrocarbon such as dichloromethane, chloroform or carbon tetrachloride; an ether such as diethyl ether, tetrahydrofuran or dioxane; a hydrocarbon such as n-hexane, benzene or toluene; an aliphatic ketone such as acetone or methyl ethyl ketone; dimethylsulfoxide; or N,N-dimethylformamide.

The above reaction can be completed in from 1 to 7 hours at a temperature within a range of from -20 ° C to the boiling point of the solvent.

Now, the present invention will be described in further detail with reference to Examples. However, it should be understood that the present invention is by no means restricted by these specific Examples.

Firstly, Examples for the preparation of the compounds of the present invention will be described.

PREPARATION EXAMPLE 1

Preparation of 9-{4-chloro-2-fluoro-5-[1-(2-methoxyethoxycarbonyl)ethylthio]phenylimino}-8-thia-1,6-diazabicyclo[4.3.0]nonan-7-one (Compound No. 2)

Into a reaction flask, 2.8 g (6 mmol) of 1,2-tetramethylene-1-{4-chloro-2-fluoro-5-[1-(2-methoxyethoxycarbonyl)ethylthio]phenylaminothiocarbonyl}hydrazine, 1.2 g (15 mmol) of pyridine and 20 ml of dichloromethane were charged, and a dichloromethane solution containing 0.8 g (8 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous sodium sulfate, then the solvent was distilled off to obtain a crude product. This crude product was purified silica gel column chromatography to obtain 1.5 g (yield: 50%) of colorless viscous substance. Refractive index $n_D^{20}$ : 1.5814.

PREPARATION EXAMPLE 2

Preparation of 9-(4-chloro-3-ethoxycarbonylmethylthiophenylimino)-8-thia-1,6-diazabicyclo[4.3.0]nonan-7-one (Compound No. 3)

Into a reaction flask, 1.2 g (3 mmol) of 1,2-tetramethylene-1-(4-chloro-3-ethoxycarbonylmethylthiophenylaminothiocarbonyl)hydrazine, 0.6 g (7 mmol) of pyridine and 20 ml of dichloromethane were charged, and a dichloromethane solution containing 0.4 g (4 mmol) of phosgene was dropwise added while

cooling the mixture with ice water. After the dropwise addition, the mixture was stirred at room temperature for 1 hour to complete the reaction. After the completion of the reaction, the reaction solution was washed with water and dried over anhydrous sodium sulfate. Then, the solvent was distilled off to obtain a crude product. This crude product was purified by silica gel column chromatography to obtain 0.6 g (yield: 47%) of colorless viscous substance. Refractive index $n_D^{20}$ : 1.6201.

PREPARATION EXAMPLE 3

Preparation of 9-(4-chloro-2-fluoro-5-propoxycarbonylmethylthiophenylimino)-8-thia-1,6-diazabicylo[4.3.0]-nonan-7-one (Compound No. 30)

Into a reaction flask, 1.6 g (3.9 mmol) of 1,2-tetrametylene-1-(4-chloro-2-fluoro-5-propoxycarbonyl methylthiophenylaminothiocarbonyl)hydrazine, 0.8 g (10 mmol) of pyridine and 20 ml of dichloromethane were charged, and a dichloromethane solution containing 0.5 g (5 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous magnesium sulfate. Then, the solvent was distilled off to obtain a crude product. This crude product was purified by silica gel column chromatography to obtain 1.0 g (yield: 59%) of colorless viscous substance. Refractive index $n_D^{20}$ : 1.6040.

PREPARATION EXAMPLE 4

Preparation of 9-(4-propargyl-7-fluoro-2H-benzoxadine-3-(4H)-on-6-ylimino)-8-thia-1,6-diazabicyclo[4.3.0]-nonan-7-one (Compound No. 18)

Into a reaction flask, 0.8 g (2 mmol) of 1,2-tetramethylene-1-(4-propargyl-7-fluoro-2H-benzoxadine-3-(4H)-on-6-ylaminothiocarbonyl)hydrazine, 0.6 g (7 mmol) of pyridine and 20 ml of dichloromethane were charged, and a dichloromethane solution containing 0.3 g (3 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous sodium sulfate. Then, the solvent was distilled off to obtain a crude product. This crude product was purified by silica gel column chromatography to obtain 0.4 g (yield: 46%) of white crystals. Melting point: 162-165 °C.

PREPARATION EXAMPLE 5

Preparation of 9-(4-chloro-2-fluoro-5-pentyloxycarbonylmethylthiophenylimino)-8-thia-1,6-diazabicyclo[4.3.0]-nonan-7-one (Compound No. 54)

Into a reaction flask, 1.6 g (3.7 mmol) of 1,2-tetramethylene-1-(4-chloro-2-fluoro-5-pentyloxycarbonylmethylthiophenylaminothiocarbonyl)hydrazine, 0.8 g (10 mmol) of pyridine and 20 ml of dichloromethane were charged, and a dichloromethane solution containing 1.5 g (5 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous magnesium sulfate. Then, the solvent was distilled off to obtain a crude product. This crude product was purified by silica gel column chromatography to obtain 1.0 g (yield: 59%) of white crystals. Melting point: 39-41 °C.

PREPARATION EXAMPLE 6

Preparation of 9-(4-chloro-3-butoxycarbonylmethylthiophenylimino)-8-thia-1,6-diazabicyclo[4.3.0]nonan-7-one (Compound No. 38)

Into a reaction flask, 1.5 g (3.7 mmol) of 1,2-tetramethylene-1-(4-chloro-3-butoxycarbonylmethylthiophenylaminothiocarbonyl)hydrazine, 0.8 g (10 mmol) of pyridine and 20 ml of dichloromethane were charged, and a dichloromethane solution containing 0.5 g (5 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous magnesium sulfate. Then, the solvent was distilled off to obtain a crude product. This crude product was purified by silica gel column chromatography to obtain 0.8 g (yield: 50%) of white crystals. Melting point: 58-60 °C.

PREPARATION EXAMPLE 7

Preparation of 9-(4-chloro-2-fluoro-5-ethoxycarbonylmethylthiophenylimino)-8-thia-1,6-diazabicyclo[4.3.0]-nonan-7-one (Compound No. 1)

Into a reaction flask, 2.0 g (5.1 mmol) of 1,2-tetramethylene-1-(4-chloro-2-fluoro-5-ethoxycarbonylmethylthiophenylaminothiocarbonyl)hydrazine, 1.0 g (12 mmol) of pyridine and 20 ml of dichloromethane were charged, and a dichloromethane solution containing 0.6 g (6 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous sodium sulfate. Then, the solvent was distilled off to obtain a crude product. This crude product was purified by silica gel column chromatography to obtain 1.3 g (yield: 61%) of colorless viscous substance. Refractive index $n_D^{20}$ : 1.5990.

PREPARATION EXAMPLE 8

Preparation of 9-(4-chloro-2-fluoro-5-methoxycarbonylmethylthiophenylimino)-8-thia-1,6-diazabicyclo[4.3.0]-nonan-7-one (Compound No. 23)

Into a reaction flask, 1.6 g (4.2 mmol) of 1,2-tetramethylene-1-(4-chloro-2-fluoro-5-methoxycarbonylmethylthiophenylaminothiocarbonyl)hydrazine, 0.8 g (10 mmol) of pyridine and 20 ml of dichloromethane were charged, and a dichloromethane solution containing 0.5 g (5 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous magnesium sulfate. Then, the solvent was distilled off to obtain a crude product. This crude product was purified by silica gel column chromatography to obtain 0.9 g (yield: 53%) of white crystals. Melting point: 99-101 °C.

PREPARATION EXAMPLE 9

Preparation of 9-(4-chloro-3-methoxycarbonylmethylthiophenylimino)-8-thia-1,6-diazabicyclo[4.3.0]nonan-7-one (Compound No. 22)

Into a reaction flask, 0.8 g (2.2 mmol) of 1,2-tetramethylene-1-(4-chloro-3-methoxycarbonylmethylthiophenylaminothiocarbonyl)hydrazine, 0.7 g (9 mmol) of pyridine and 20 ml of dichloromethane were charged, and a dichloromethane solution containing 0.4 g (4 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous magnesium sulfate. Then, the solvent was distilled off to obtain a crude product. This crude product was purified by column chromatography to obtain 0.6 g (yield: 71%) of colorless viscous substance. Refractive index $n_D^{20}$ : 1.6250.

PREPARATION EXAMPLE 10

Preparation of 9-[4-chloro-3-(1-pentyloxycarbonylethylthio)phenylimino]-8-thia-1,6-diazabicyclo[4.3.0]nonan-7-one (Compound No. 97)

Into a reaction flask, 1.6 g (3.7 mmol) of 1,2-tetramethylene-1-[4-chloro-3-(1-pentyloxycarbonylethyl thio)phenylaminothiocarbonyl]hydrazine, 0.8 g (10 mmol) of pyridine and 20 ml of dichloromethane, were charged, and a dichloromethane solution containing 0.5 g (5 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous sodium sulfate, and then the solvent was distilled off to obtain a crude product. This crude product was purified by silica gel column chromatography to obtain 1.1 g (yield: 65%) of colorless viscous substance. Refractive index $n_D^{20}$ : 1.5820.

PREPARATION EXAMPLE 11

Preparation of 9-(2,4-difluoro-5-ethoxycarbonylphenylimino)-8-thia-1,6-diazabicyclo[4.3.0]nonane-7-thione (Compound No. 82)

Into a reaction flask, 4.0 g (12 mmol) of 1,2-tetramethylene-1-(2,4-difluoro-5-ethoxycarbonylphenylaminothiocarbonyl)hydrazine, 2.4 g (30 mmol) of pyridine and 20 ml of dichloromethane, were

charged, and a dichloromethane solution containing 1.5 g (15 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous magnesium sulfate, and then the solvent was distilled off to obtain a crude product. This crude product was purified by silica gel column chromatography to obtain 2.3 g (yield: 58%) of white crystals. Melting point: 91-92 °C.

PREPARATION EXAMPLE 12

Preparation of 9-(2,4-dichloro-5-ethoxycarbonylphenylimino)-8-thia-1,6-diazabicyclo[4.3.0]nonan-7-one (Compound No. 80)

Into a reaction flask, 2.7 g (25 mmol) of 1,2-tetramethylene-1-(2,4-dichloro-5-ethoxycarbonylphenylaminothiocarbonyl)hydrazine, 1.5 g (19 mmol) of pyridine and 20 ml of dichloromethane, were charged, and a dichloromethane solution containing 1.0 g of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous sodium sulfate, and then the solvent was distilled off to obtain a crude product. This crude product was purified by silica gel column chromatography to obtain 1.7 g (yield: 59%) of white crystals. Melting point: 81-83 °C.

PREPARATION EXAMPLE 13

Preparation of 9-(2,4-difluoro-5-isopropoxycarbonylphenylimino)-8-thia-1,6-diazabicyclo[4.3.0]nonan-7-one (Compound No. 84)

Into a reaction flask, 3.0 g (8.7 mmol) of 1,2-tetramethylene-1-(2,4-difluoro-5-isopropoxycarbonylphenylaminothiocarbonyl)hydrazine, 1.7 g (22 mmol) of pyridine and 20 ml of dichloromethane, were charged, and a dichloromethane solution containing 1.1 g (11 mmol) of phosgene was dropwise added while cooling the mixture with ice water. After the dropwise addition, the mixture was stirred at room temperature for 1 hour to complete the reaction. After the completion of the reaction, the reaction solution was washed with water and dried over anhydrous sodium sulfate, and then the solvent was distilled off to obtain a crude product. This crude product was purified by silica gel column chromatography to obtain 1.7 g (yield: 53%) of white crystals. Melting point: 86-88 °C.

The 9-phenylimino-8-thia-1,6-diazabicyclo[4.3.0] nonan-7-one derivative of the formula I is useful as an active ingredient for a herbicide. When the compound of the formula I of the present invention is used as a herbicide for a paddy rice field, an upland field, an orchard or a non-agricultural field, the active ingredient can be used in a suitable formulation depending upon the particular purpose. Usually, the active ingredient is diluted with an inert liquid or solid carrier, and used in the form of a formulation such as a dust, a wettable powder, an emulsifiable concentrate, a granule, etc., if necessary by adding a surfactant and other additives. Further, the compound of the present invention may be used in combination with an insecticide, a nematocide, a fungicide, other herbicides, a plant growth controlling agent, a fertilizer, etc., as the case requires.

Now, the formulations will be described in detail with reference to typical Formulation Examples. In the following Formulation Examples, "parts" means "parts by weight".

FORMULATION EXAMPLE 1 Wettable powder

10.0 parts of Compound No. 1, 0.5 part of Emulgen (trademark of Kao Corporation) 810, 0.5 part of Demol (trademark of Kao Corporation) N, 20.0 parts of Kunilite (trademark of Kunimine Industries Co., Ltd.) 201, and 69.0 parts of Zeeklite (trademark of Zeeklite Co., Ltd.) CA, were mixed and pulverized to obtain a wettable powder containing 10% of an active ingredient.

FORMULATION EXAMPLE 2 Wettable powder

10.0 parts of Compound No. 2, 0.5 part of Emulgen 810, 0.5 part of Demol N, 20.0 parts of Kunilite 201, 5.0 parts of Carplex 80 and 64.0 parts of Zeeklite CA, were mixed and pulverized to obtain a wettable powder containing 10% of the active ingredient.

FORMULATION EXAMPLE 3 Emulsifiable concentrate

To 30 parts of Compound No. 3, 60 parts of a mixture of xylene and isophorone in equal amounts and 10 parts of surfactant Sorpol (trademark of Toho Chemical Industry Co., Ltd.) 800A, were added, and the mixture was thoroughly mixed to obtain 100 parts of an emulsifiable concentrate.

FORMULATION EXAMPLE 4 Granules

10 parts of water was added to 10 parts of Compound No. 4, 80 parts of a filler obtained by mixing talc and bentonite in a ratio of 1 : 3, 5 parts of white carbon and 5 parts of surfactant Sorpol 800A, and the mixture was thoroughly kneaded to obtain a paste, which was extruded from sieve openings having a diameter of 0.7 mm and dried, and then cut into a length of from 0.5 to 1 mm, to obtain 100 parts of granules.

The compounds of the formula I of the present invention exhibit excellent herbicidal effects at a very low dose in a wide range from the germination stage to the growing stage of annual weeds such as barnyardgrass (Echinochloa crus-galli), umbrella-plant (Cyperus difformis L.), monochoria (Monochoria vaginalis Presl), spike-flowered rotala (Rotala indica Koehne), false pimpernel (Lindernia procumbens Philcox) and Dopatrium junceum Hamilt, and perennial weeds such as bulrush (Scirpus juncoides Roxb.), slender spikerush (Eleocharis acicularis Roem. et Schult.), water-plantain (Alisma canaliculatum A. Br. et Bouche), sagittaria (Sagittaria pygmaea Miq.) and cyperus sp. (Cyperus serotinus Rottb.) which grow in paddy fields. At the same time, they have high selectivity for paddy field rice. Further, they exhibit high herbicidal effects, by soil treatment or by foliage treatment, against various weeds in the upland fields, for example, broad leaf weeds such as smart weed (Polygonum nodosum L.), pigweed (Amaranthus retroflexus), lambsquaters (Chenopodium album), speed-well (Veronica persica), wild mustard (Brassica kaber var. pinnatifida), cocklebur (Xanthium strumarium), morningglory (Ipomoea spp), hemp sesbania (Sesbania exaltata Raf. ) and velvetleaf (Abtilon theophrasti), cyperaceous weeds such as rice flatsedge (Cyperus iria L.), and gramineous weeds such as barnyardgrass, large crabgrass (Digitaria sanguinalis) and green foxtail (Setaria viridis). At the same time, they have a feature that they are highly safe to crop plants such as upland rice, wheat, soybean and corn.

The dose of the compound of the present invention is usually within a range of from 1 g to 10 kg/ha. More specifically, the dose is usually from 5 g to 5 kg/ha for upland fields, from 10 g to 1 kg/ha for paddy rice fields, and from 200 g to 5 kg/ha for non-agricultural fields.

Further, the compounds of the present invention have excellent residual effects, and show stabilized effects for a long period of time also in paddy fields. They are also useful for orchard, grassland, lawn and non-agricultural fields.

Now, the herbicidal effects of the herbicides of the present invention will be described with reference to Test Examples.

TEST EXAMPLE 1 Herbicidal test by soil treatment of paddy field

Into a porcelain pot having a diameter of 10 cm, paddy field soil was filled and puddled. Then, seeds of barnyardgrass, umbrella plant, monochoria and bulrush were sown, and water was introduced to a depth of 3 cm.

Next day, the wettable powder prepared in accordance with Formulation Example 1, was diluted with water and dropwise applied to the surface of the water. The amount of the active ingredient applied, was 400 g/10a. Then, the pot was left in a green house. Twenty one days after the application, the herbicidal effects were evaluated in accordance with the standards identified in Table 2. The results are shown in Tables 3, 4 and 5.

Table 2

| Index | Herbicidal effects and phytotoxicity |
|---|---|
| 5 | Withered |
| 4.5 | Herbicidal effect (or phytotoxicity) in a range of 90 to 99% |
| 4 | Herbicidal effect (or phytotoxicity) in a range of 80 to 89% |
| 3.5 | Herbicidal effect (or phytotoxicity) in a range of 70 to 79% |
| 3 | Herbicidal effect (or phytotoxicity) in a range of 60 to 69% |
| 2.5 | Herbicidal effect (or phytotoxicity) in a range of 50 to 59% |
| 2 | Herbicidal effect (or phytotoxicity) in a range of 40 to 49% |
| 1.5 | Herbicidal effect (or phytotoxicity) in a range of 30 to 39% |
| 1 | Herbicidal effect (or phytotoxicity) in a range of 20 to 29% |
| 0.5 | Herbicidal effect (or phytotoxicity) in a range of 1 to 19% |
| 0 | No herbicidal effect (or no phytotoxicity) |

Table 3

| Compound No. | Herbicidal effects | | | |
|---|---|---|---|---|
| | Barnyard-grass | Umbrella plant | Monochoria | Bulrush |
| 1 | 5 | 5 | 5 | 5 |
| 2 | 5 | 5 | 5 | 5 |
| 18 | 5 | 5 | 5 | 5 |
| 19 | 5 | 5 | 5 | 5 |
| 20 | 5 | 5 | 5 | 5 |
| 21 | 5 | 5 | 5 | 5 |

Table 4

| Compound No. | Herbicidal effects | | | |
|---|---|---|---|---|
| | Barnyard-grass | Umbrella plant | Monochoria | Bulrush |
| 80 | 5 | 5 | 5 | 5 |
| 81 | 5 | 5 | 5 | 5 |
| 82 | 5 | 5 | 5 | 5 |
| 83 | 5 | 5 | 5 | 5 |
| 84 | 5 | 5 | 5 | 5 |

Table 5

| Compound No. | Herbicidal effects | | | |
|---|---|---|---|---|
| | Barnyard-grass | Umbrella plant | Monochoria | Bulrush |
| 85 | 5 | 5 | 5 | 5 |
| 86 | 5 | 5 | 5 | 5 |
| 87 | 5 | 5 | 5 | 5 |
| 88 | 5 | 5 | 5 | 5 |
| 89 | 5 | 5 | 5 | 5 |
| 90 | 5 | 5 | 5 | 5 |
| 91 | 5 | 5 | 5 | 5 |
| 92 | 5 | 5 | 5 | 5 |
| 93 | 5 | 5 | 5 | 5 |
| 94 | 5 | 5 | 5 | 5 |
| 96 | 5 | 5 | 5 | 5 |
| 97 | 5 | 5 | 5 | 5 |
| 98 | 5 | 5 | 5 | 5 |
| 99 | 5 | 5 | 5 | 5 |
| 100 | 5 | 5 | 5 | 5 |

Likewise, Compound Nos. 3-17 and 22-67 completely killed the test weeds.

TEST EXAMPLE 2 The herbicidal test in soil teatment of upland field

Into a 120 $cm^2$ plastic pot, upland field soil was filled, and seeds of barnyardgrass, large crabgrass, smart weed, pigweed and rice flatsedge were sown and covered with soil.

A wettable powder of each test compound formulated in accordance with Formulation Example 1, was diluted with water in an amount of 100 liter/10a and uniformly applied to the surface of soil by means of a small size spray at a dose of 400 g/10a of the active ingredient. After the application, the pot was left for 21 days in a green house, and then the herbicidal effects were evaluated in accordance with the standards identified in Table 2. The results are shown in Tables 6, 7 and 8.

Table 6

| Compound No. | Herdbicidal effects | | | | |
|---|---|---|---|---|---|
| | Barnyard-grass | Large crabgrass | Smart-weed | Pigweed | Rice flatsedge |
| 1 | 5 | 5 | 5 | 5 | 5 |

Table 7

| Compound No. | Herdbicidal effects | | | | |
|---|---|---|---|---|---|
| | Barnyard-grass | Large crabgrass | Smart-weed | Pigweed | Rice flatsedge |
| 2 | 5 | 5 | 5 | 5 | 5 |
| 6 | 5 | 5 | 5 | 5 | 5 |
| 7 | 5 | 5 | 5 | 5 | 5 |
| 17 | 5 | 5 | 5 | 5 | 5 |
| 18 | 5 | 5 | 5 | 5 | 5 |
| 20 | 5 | 5 | 5 | 5 | 5 |
| 21 | 5 | 5 | 5 | 5 | 5 |
| 31 | 5 | 5 | 5 | 5 | 5 |
| 39 | 4 | 5 | 5 | 5 | 5 |
| 44 | 5 | 5 | 5 | 5 | 5 |
| 50 | 5 | 5 | 4 | 5 | 5 |
| 59 | 4 | 5 | 5 | 5 | 5 |
| 63 | 5 | 5 | 5 | 5 | 5 |
| 66 | 5 | 5 | 5 | 5 | 5 |

Table 8

| Compound No. | Herdbicidal effects | | | | |
|---|---|---|---|---|---|
| | Barnyard-grass | Large crabgrass | Smart-weed | Pigweed | Rice flatsedge |
| 80 | 5 | 5 | 5 | 5 | 5 |
| 84 | 4 | 5 | 5 | 5 | 5 |
| 85 | 5 | 5 | 5 | 5 | 5 |
| 86 | 5 | 5 | 5 | 5 | 5 |
| 88 | 5 | 5 | 5 | 5 | 5 |
| 89 | 4 | 5 | 5 | 5 | 5 |
| 92 | 5 | 5 | 5 | 5 | 5 |
| 93 | 5 | 5 | 4 | 5 | 5 |
| 94 | 5 | 5 | 5 | 5 | 5 |
| 100 | 5 | 5 | 5 | 5 | 5 |

TEST EXAMPLE 3 The herbicidal test in foliage treatment in upland field

Into a 120 $cm^2$ plastic pot, upland field soil was filled, and seeds of barnyardgrass, large crabgrass, smart weed, pigweed, lambsquaters and rice flatsedge, were sown, and grown in a green house until barnyardgrass grew to the 3 leaf stage. When barnyardgrass reached the 3 leaf stage, a wettable powder of each test compound formulated in accordance with Formulation Example 1 was diluted with water in an amount of 100 liter/10a and applied to the foliage of the plants from above by a small size spray at a dose of 400 g/10a of the active ingredient. After the application, the pot was left for 21 days in a green house, and then the herbicidal effects were evaluated in accordance with the standards identified in Table 2. The results are shown in Tables 9, 10 and 11.

Table 9

| Compound No. | Herbicidal effects | | | | | |
|---|---|---|---|---|---|---|
| | Barnyard-grass | Large crabgrass | Smart-weed | Pigweed | Lambs-quaters | Rice flatsedge |
| 1 | 5 | 5 | 5 | 5 | 5 | 5 |

Table 10

| Compound No. | Herbicidal effects | | | | | |
|---|---|---|---|---|---|---|
| | Barnyard-grass | Large crabgrass | Smart-weed | Pigweed | Lambs-quaters | Rice flatsedge |
| 2 | 5 | 5 | 5 | 5 | 5 | 5 |
| 3 | 5 | 5 | 5 | 5 | 5 | 5 |
| 5 | 5 | 5 | 5 | 5 | 5 | 5 |
| 6 | 5 | 5 | 5 | 5 | 5 | 5 |
| 7 | 5 | 5 | 5 | 5 | 5 | 5 |
| 8 | 5 | 5 | 5 | 5 | 5 | 5 |
| 10 | 5 | 5 | 5 | 5 | 5 | 5 |
| 11 | 5 | 5 | 5 | 5 | 5 | 5 |
| 16 | 5 | 5 | 5 | 5 | 5 | 5 |
| 17 | 5 | 5 | 5 | 5 | 5 | 5 |
| 18 | 5 | 5 | 5 | 5 | 5 | 5 |
| 19 | 5 | 5 | 5 | 5 | 5 | 5 |
| 20 | 5 | 5 | 5 | 5 | 5 | 5 |
| 21 | 5 | 5 | 5 | 5 | 5 | 5 |
| 25 | 5 | 5 | 5 | 5 | 5 | 5 |
| 31 | 5 | 5 | 5 | 5 | 5 | 5 |
| 35 | 5 | 5 | 5 | 5 | 5 | 5 |
| 39 | 5 | 5 | 5 | 5 | 5 | 5 |
| 40 | 5 | 5 | 5 | 5 | 5 | 5 |
| 41 | 5 | 5 | 5 | 5 | 5 | 5 |
| 44 | 5 | 5 | 5 | 5 | 5 | 5 |
| 49 | 5 | 5 | 5 | 5 | 5 | 5 |
| 50 | 5 | 5 | 5 | 5 | 5 | 5 |
| 54 | 5 | 5 | 5 | 5 | 5 | 5 |
| 61 | 5 | 5 | 5 | 5 | 5 | 5 |
| 63 | 5 | 5 | 5 | 5 | 5 | 5 |
| 66 | 5 | 5 | 5 | 5 | 5 | 5 |
| 67 | 5 | 5 | 5 | 5 | 5 | 5 |

Table 11

| Compound No. | Herbicidal effects | | | | | |
|---|---|---|---|---|---|---|
| | Barnyard-grass | Large crabgrass | Smart-weed | Pigweed | Lambs-quaters | Rice flatsedge |
| 80 | 5 | 5 | 5 | 5 | 5 | 5 |
| 81 | 5 | 5 | 5 | 5 | 5 | 5 |
| 82 | 5 | 5 | 5 | 5 | 5 | 5 |
| 83 | 5 | 5 | 5 | 5 | 5 | 5 |
| 84 | 5 | 5 | 5 | 5 | 5 | 5 |
| 85 | 5 | 5 | 5 | 5 | 5 | 5 |
| 86 | 5 | 5 | 5 | 5 | 5 | 5 |
| 87 | 5 | 5 | 5 | 5 | 5 | 5 |
| 88 | 5 | 5 | 5 | 5 | 5 | 5 |
| 89 | 5 | 5 | 5 | 5 | 5 | 5 |
| 90 | 5 | 5 | 5 | 5 | 5 | 5 |
| 91 | 5 | 5 | 5 | 5 | 5 | 5 |
| 92 | 5 | 5 | 5 | 5 | 5 | 5 |
| 93 | 5 | 5 | 5 | 5 | 5 | 5 |
| 94 | 5 | 5 | 5 | 5 | 5 | 5 |
| 95 | 5 | 5 | 5 | 5 | 5 | 5 |
| 96 | 5 | 5 | 5 | 5 | 5 | 5 |
| 97 | 5 | 4 | 5 | 5 | 5 | 5 |
| 98 | 5 | 5 | 5 | 5 | 5 | 5 |
| 99 | 5 | 5 | 5 | 5 | 5 | 5 |
| 100 | 5 | 5 | 5 | 5 | 5 | 5 |

TEST EXAMPLE 4 The herbicidal and phytotoxicity tests in soil treatment of paddy field

Into a 1/5,000a Wagner pot, paddy field soil was filled and puddled, and water was introduced thereto. In the pot, three germinated tubers of sagittaria were embended in the surface layer of the soil, and seeds of water-plantain were sown in the surface layer of the soil. Further, two rice plants of 2.5 leaf stage were transplanted in a depth of 2 cm. Then, water was introduced to a depth of 3 cm. Next day, a prescribed amount of wettable powder formulated in accordance with Formulation Example 1, was diluted with water and dropwise applied to the water surface. Then, the pot was cultivated in a green house, and 30 days after the application, the herbicidal effect and phytotoxicity were evaluated in accordance with the standards identified in Table 2. The results are shown in Tables 12 and 13.

Table 12

| Compound No. | Dose of active ingredients (g/10a) | Herbicidal effects | | Phytotoxicity |
|---|---|---|---|---|
| | | Water-plantain | Sagittaria | Transplanted paddy field rice |
| 1 | 12.5 | 5 | 5 | 0.5 |
| 3 | 6.3 | 5 | 5 | 0 |
| 6 | 12.5 | 5 | 5 | 0.5 |
| 10 | 12.5 | 5 | 5 | 0 |
| 17 | 6.3 | 5 | 5 | 0 |
| 24 | 25 | 5 | 5 | 0 |
| 25 | 25 | 5 | 5 | 0 |
| 26 | 12.5 | 5 | 5 | 0 |
| 27 | 12.5 | 5 | 5 | 0 |
| 28 | 12.5 | 5 | 4 | 0 |
| 31 | 6.3 | 5 | 5 | 0 |
| 32 | 25 | 5 | 5 | 0 |
| 35 | 25 | 5 | 5 | 0 |
| 40 | 25 | 5 | 5 | 0 |
| 41 | 12.5 | 5 | 5 | 0 |
| 46 | 25 | 5 | 5 | 0 |
| 51 | 12.5 | 5 | 5 | 0 |
| 54 | 12.5 | 5 | 5 | 0 |
| 55 | 12.5 | 5 | 5 | 0 |
| 56 | 12.5 | 5 | 5 | 0 |
| 64 | 50 | 5 | 5 | 0 |
| 67 | 12.5 | 5 | 5 | 0 |
| Oxa-diazon | 50 | 5 | 5 | 2 |
| | 25 | 5 | 2 | 2 |
| | 12.5 | 5 | 1 | 1 |

Table 13

| Compound No. | Dose of active ingredients (g/10a) | Herbicidal effects | | Phytotoxicity |
|---|---|---|---|---|
| | | Water-plantain | Sagittaria | Transplanted paddy field rice |
| 80 | 6.3 | 5 | 4 | 1.0 |
| 85 | 12.5 | 5 | 5 | 0 |
| 87 | 25 | 5 | 5 | 0 |
| 89 | 12.5 | 5 | 4 | 0 |
| 91 | 25 | 5 | 5 | 0 |
| 93 | 25 | 5 | 5 | 0 |
| 97 | 25 | 5 | 5 | 0 |
| 99 | 12.5 | 5 | 4 | 0.5 |
| Oxa-diazon | 50 | 5 | 5 | 2.0 |
| | 25 | 5 | 2 | 2.0 |
| | 12.5 | 5 | 1 | 1.0 |

TEST EXAMPLE 5 Selectivity test for soybean

Into a 120 cm$^2$ plastic pot, upland field soil was filled, and seeds of soybean, cocklebur, and morning-glory were sown. After cultivating in a green house for 14 days, a prescribed amount of a wettable powder formulated in accordance with Formulation Example 1 was diluted with water in an amount of 100 liter/10a and applied to the foliage of the plants from above by a small size spray. After the application, the pot was left in a green house for 21 days, and then the herbicidal effects and phytotoxicity were evaluated in accordance with the standards identified in Table 2. The results are shown in Tables 14 and 15.

Table 14

| Compound No. | Dose of active ingredient (g/10a) | Herbicidal effect | | Phytotoxicity |
|---|---|---|---|---|
| | | Cocklebur | Morning-glory | Soybean |
| 1 | 3 | 5 | 4 | 0 |
| 3 | 3 | 5 | - | 0.5 |
| 7 | 10 | 5 | - | 0.5 |
| 22 | 10 | 5 | 4 | 0 |
| 23 | 10 | 5 | 5 | 0 |
| 24 | 3 | 5 | - | 0 |
| 30 | 1 | 5 | 4 | 0 |
| 31 | 3 | 5 | - | 0 |
| 33 | 3 | 5 | - | 0 |
| 34 | 10 | 5 | 5 | 0.5 |
| 38 | 3 | 5 | - | 0 |
| 39 | 1 | 5 | - | 0 |
| 40 | 3 | 5 | - | 0 |
| 41 | 3 | 4 | - | 0.5 |
| 44 | 1 | 5 | - | 0 |
| 48 | 10 | 4.5 | - | 0 |
| 49 | 3 | 5 | 5 | 0.5 |
| 50 | 10 | 5 | 5 | 0.5 |
| 54 | 3 | 5 | 5 | 0.5 |
| 55 | 3 | 5 | - | 0 |
| 56 | 3 | 4.5 | - | 0 |
| 63 | 1 | 5 | 5 | 0 |
| 64 | 10 | 5 | - | 0 |
| 65 | 10 | 5 | 4 | 0 |
| 66 | 3 | 5 | 5 | 0.5 |
| 67 | 10 | 5 | - | 0.5 |
| Oxa-diazon | 50 | 3 | 2 | 1 |
| | 25 | 2 | 1 | 0.5 |
| | 6.3 | 0 | 0.5 | 0 |

Table 15

| Compound No. | Dose of active ingredient (g/10a) | Herbicidal effect | | Phytotoxicity |
|---|---|---|---|---|
| | | Cocklebur | Morning-glory | Soybean |
| 85 | 1 | 4.5 | 4 | 0.5 |
| 87 | 3 | 5 | 5 | 1 |
| 88 | 1 | 5 | 4 | 0 |
| 90 | 3 | 5 | 5 | 0.5 |
| 92 | 3 | 5 | 4.5 | 0 |
| 93 | 3 | 5 | 4 | 0 |
| 94 | 3 | 5 | 4.5 | 0 |
| 95 | 10 | 5 | 4 | 0 |
| 96 | 1 | 4.5 | 4 | 0 |
| 97 | 3 | 4.5 | 5 | 0 |
| 98 | 3 | 5 | 5 | 0 |
| 100 | 3 | 5 | 4.5 | 0 |
| Oxa-diazon | 50 | 3 | 2 | 1 |
| | 25 | 2 | 1 | 1 |
| | 6.3 | 0 | 0.5 | 0.5 |
| | 3 | 0 | 0 | 0 |

TEST EXAMPLE 6 Herbicidal and phytotoxicity test in the foliage treatment of upland field

Into a 330 $cm^2$ plastic pot, upland field soil was filled, and seeds of wheat, corn, pigweed, lambsquaters and velvetleaf were sown and grown in a green house until the corn grew to the 4 leaf stage. When the corn reached the 4 leaf stage, a prescribed amount of a wettable powder formulated in accordance with Formulation Example 1 was diluted with water in an amount of 100 liter/10a and applied to the foliage of the plants from above by a small size spray. After the application, the pot was left in a green house for 21 days, and then the herbicidal effects and phytotoxicity were evalutated in accordance with the standards identified in Table 2. The results a shown in Tables 16 and 17.

Table 16

| Compound No. | Dose of active ingredient (g/10a) | Herbicidal effect | | | Phyto-toxicity |
|---|---|---|---|---|---|
| | | Pigweed | Lambs-quaters | Velvet-leaf | Corn |
| 1 | 1.6 | 5 | 5 | 5 | 0 |
| 3 | 1.6 | 5 | 5 | 5 | 0 |
| 4 | 1.6 | 5 | 5 | 5 | 0 |
| 5 | 1.6 | 5 | 5 | 5 | 0.5 |
| 8 | 1.6 | 4.5 | 4.5 | 5 | 0.5 |
| 10 | 1.6 | 5 | 5 | 5 | 0 |
| 11 | 1.6 | 5 | 5 | 5 | 0 |
| 12 | 1.6 | 5 | 5 | 5 | 0.5 |
| 13 | 1.6 | 5 | 5 | 5 | 0 |
| 14 | 1.6 | 5 | 5 | 5 | 0 |
| 16 | 1.6 | 5 | 5 | 5 | 0 |
| 19 | 1.6 | 5 | 5 | 5 | 1.5 |
| 23 | 1.6 | 5 | 5 | 5 | 0 |
| 30 | 1.6 | 5 | 5 | 5 | 0 |
| 34 | 1.6 | 5 | 5 | 5 | 0 |
| 39 | 1.6 | 5 | 5 | 5 | 0 |
| 61 | 1.6 | 5 | 5 | 4.5 | 0.5 |
| 62 | 1.6 | 5 | 5 | 5 | 0 |
| 63 | 1.6 | 5 | 5 | 5 | 0.5 |
| 72 | 1.6 | 5 | 4.5 | 5 | 0.5 |
| 74 | 1.6 | 5 | 5 | 5 | 0 |
| 77 | 1.6 | 5 | 5 | 5 | 0 |
| Oxa-diazon | 25 | 4.5 | 4.5 | 5 | 1.5 |
| | 6.3 | 2 | 3.5 | 4.5 | 1 |
| | 1.6 | 0.5 | 1 | 2.5 | 0 |

Table 17

| Compound No. | Dose of active ingredient (g/10a) | Herbicidal effect | | | Phyto-toxicity | |
|---|---|---|---|---|---|---|
| | | Pigweed | Lambs-quaters | Velvet-leaf | Wheat | Corn |
| 85 | 0.4 | 5 | 5 | 5 | 0.5 | 0 |
| 86 | 1.6 | 5 | 5 | 5 | 0 | 0 |
| 87 | 0.4 | 5 | 5 | 5 | 0 | 0 |
| 89 | 1.6 | 5 | 5 | 5 | 0.5 | 0 |
| 91 | 1.6 | 5 | 5 | 5 | 0.5 | 0.5 |
| 95 | 0 4 | 5 | 5 | 5 | 0 | 0.5 |
| 97 | 0.4 | 5 | 5 | 5 | 0 | 0 |
| 98 | 0.4 | 5 | 4.5 | 5 | 0 | 0 |
| 99 | 1.6 | 5 | 5 | 5 | 0 | 0.5 |
| 100 | 0.4 | 5 | 5 | 5 | 0 | 0 |
| Oxa-diazon | 25 | 4.5 | 4.5 | 5 | 1.5 | 1.5 |
| | 6.3 | 2 | 3.5 | 4.5 | 1 | 1 |
| | 1.6 | 0.5 | 1 | 2.5 | 0 | 0 |

**Claims**

**Claims for the following Contracting States : BE, CH, DE, FR, GB, IT, LI, NL**

**1.** A 9-phenylimino-8-thia-1,6-diazabicyclo [4.3.0]nonane-7-one derivative having the formula:

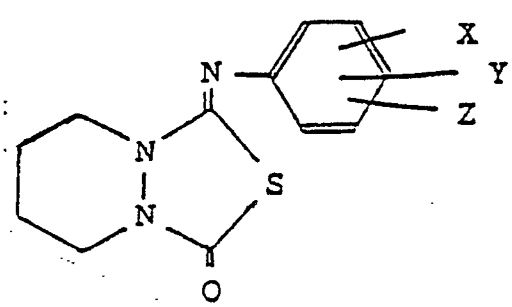

(I)

wherein each of X and Y is hydrogen or halogen, and Z is

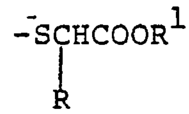

wherein R is hydrogen or alkyl, and $R^1$ is alkyl, cycloalkyl or alkoxyalkyl, or -COOQ wherein Q is alkyl, or Y and Z together form

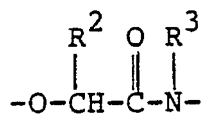

bonded to the phenyl ring, wherein $R^2$ is hydrogen or alkyl, and $R^3$ is alkyl, alkenyl or alkynyl.

**2.** The compound according to Claim 1, wherein Z is

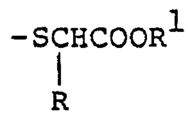

wherein R and $R^1$ are as defined in Claim 1.

**3.** The compound according to Claim 1, wherein Z is $-SCH_2COOR^1$ wherein $R^1$ is as defined in Claim 1.

**4.** The compound according to Claim 1, wherein Z is

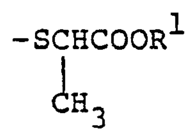

wherein $R^1$ is as defined in Claim 1.

**5.** The compound according to Claim 1, which has the formula:

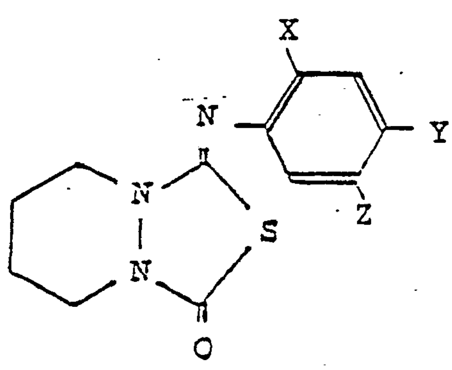

wherein X, Y and Z are as defined in Claim 1.

**6.** The compound according to Claim 5 wherein Z is

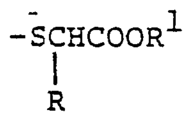

wherein R and $R^1$ are as defined in Claim 1.

7. The compound according to Claim 6, wherein R is hydrogen or methyl.

8. The compound according to any one of Claims 1 to 7 wherein $R^1$ is alkyl.

9. The compound according to any one of Claims 1 to 8 wherein X is hydrogen or halogen and Y is halogen.

10. The compound according to any one of Claims 1 to 8 wherein X is hydrogen or fluorine, and Y is chlorine.

11. A herbicidal composition comprising a herbicidally effective amount of a compound of the formula I as defined in Claim 1 and a carrier.

**Claims for the following Contracting State : ES**

1. A process for preparing a 9-phenylimino-8-thia-1,6-diazabicyclo[4.3.0]nonane-7-one derivate having the formula:

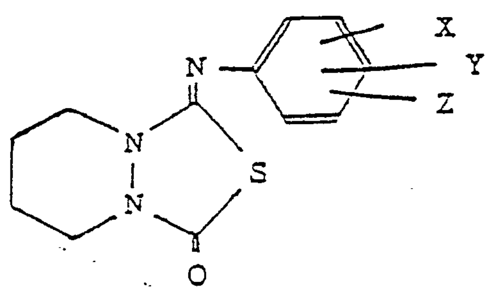

(I)

wherein each of X and Y is hydrogen or halogen, and Z is

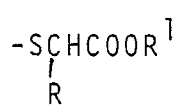

wherein R is hydrogen or alkyl, and $R^1$ is alkyl, cycloalkyl or alkoxyalkyl, or -COOQ wherein Q is alkyl, or Y and Z together from

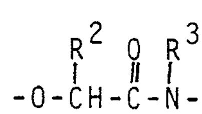

bonded to the phenyl ring, wherein $R^2$ is hydrogen or alkyl, and $R^3$ is alkyl, alkenyl or alkynyl, which comprises reacting a compound having the general formula:

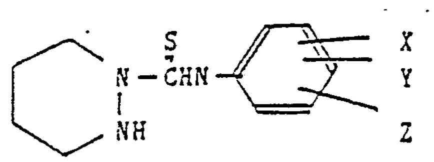

( II )

wherein X, Y and Z are as defined above with the compound of the formula (III)

$COCl_2$ (III)

in the presence or absence of a base.

2. The process according to Claim 1, wherein Z is

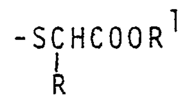

wherein R and $R^1$ are as defined in Claim 1.

3. The process according to Claim 1, wherein Z is $-SCH_2COOR^1$ wherein $R^1$ is as defined in Claim 1.

4. The process acccording to Claim 1, wherein Z is

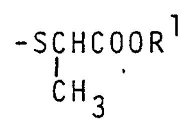

wherein $R^1$ is as defined in Claim 1.

5. The process according to Claim 1, which produces a compound having the following formula:

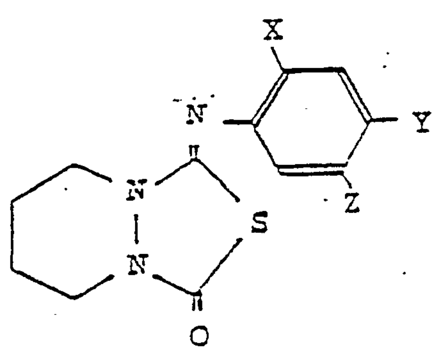

wherein X, Y and Z are as defined in Claim 1.

6. The process according to Claim 5, wherein Z is

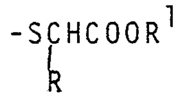

wherein R and $R^1$ are as defined in Claim 1.

**7.** The process according to Claim 6, wherein R is hydrogen or methyl.

**8.** The process according to any one of Claims 1 to 7 wherein $R^1$ is alkyl.

**9.** The process according to any one of Claims 1 to 8 wherein X is hydrogen or halogen and Y is halogen.

**10.** The process according to any one of Claims 1 to 8 wherein X is hydrogen or fluorine, and Y is chlorine.

**11.** A herbicidal composition comprising a herbicidally effective amount of a compound of the formula I as defined in Claim 1 and a carrier.

## Revendications

**Revendications pour les Etats contractants suivants : BE, CH, DE, FR, GB, IT, LI, NL**

**1.** Dérivé de phénylimino-9 thia-8 diaza-1,6 bicyclo[4.3.0]nonanone-7, représenté par la formule :

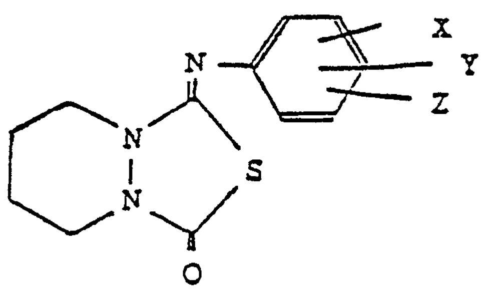

(I)

dans laquelle :
- X et Y représentent chacun hydrogène ou halogène ; et
- Z représente

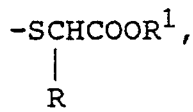

où
- R représente hydrogène ou alkyle ; et
- $R^1$ représente alkyle, cycloalkyle ou alcoxyalkyle, ou -COOQ, où Q représente alkyle ; ou
- Y et Z forment ensemble

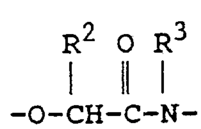

relié au noyau phényle, où $R^2$ représente hydrogène ou alkyle, et $R^3$ représente alkyle, alcényle ou alcynyle.

2. Composé selon la revendication 1, dans lequel Z représente

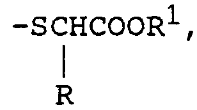

où R et $R^1$ sont tels que définis à la revendication 1.

3. Composé selon la revendication 1, dans lequel Z représente $-SCH_2COOR^1$, où $R^1$ est tel que défini à la revendication 1.

4. Composé selon la revendication 1, dans lequel Z représente

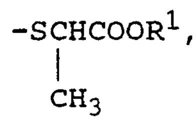

où $R^1$ est tel que défini à la revendication 1.

5. Composé selon la revendication 1, qui est représenté par la formule :

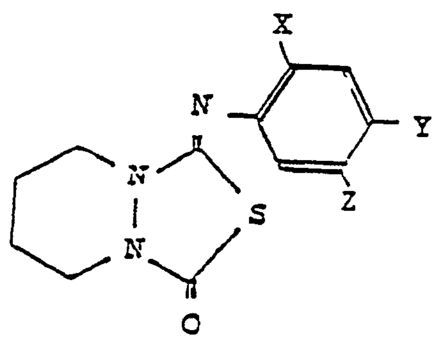

dans laquelle X, Y et Z sont tels que définis à la revendication 1.

6. Composé selon la revendication 5, dans lequel Z représente

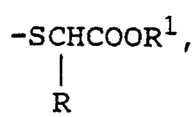

où R et $R^1$ sont tels que définis à la revendication 1.

7. Composé selon la revendication 6, dans lequel R représente hydrogène ou méthyle.

8. Composé selon l'une des revendications 1 à 7, dans lequel $R^1$ représente alkyle.

9. Composé selon l'une des revendications 1 à 8, dans lequel X représente hydrogène ou halogène et Y représente halogène.

10. Composé selon l'une des revendications 1 à 8, dans lequel X représente hydrogène ou fluor, et Y représente chlore.

11. Composition herbicide comprenant une quantité efficace pour détruire les mauvaises herbes d'un

composé de la formule (I), tel que défini à la revendication 1, et un support.

**Revendications pour l'Etat contractant suivant : ES**

1. Procédé de fabrication d'un dérivé de phénylimino-9 thia-8 diaza-1,6 bicyclo[4.3.0]nonanone-7, représenté par la formule :

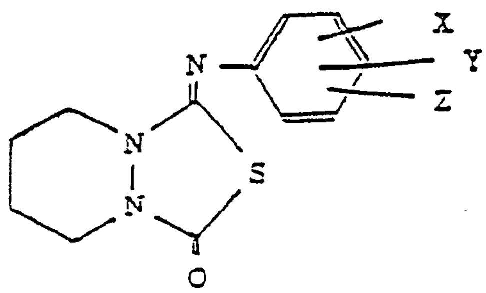

(I)

dans laquelle :
- X et Y représentent chacun hydrogène ou halogène ; et
- Z représente

$$-S\underset{\displaystyle R}{\underset{|}{C}}HCOOR^1,$$

où
- R représente hydrogène ou alkyle ; et
- $R^1$ représente alkyle, cycloalkyle ou alcoxyalkyle,

ou -COOQ, où Q représente alkyle ; ou
- Y et Z forment ensemble

$$-O-\overset{R^2}{\overset{|}{CH}}-\overset{O}{\overset{\|}{C}}-\overset{R^3}{\overset{|}{N}}-$$

relié au noyau phényle, où $R^2$ représente hydrogène ou alkyle, et $R^3$ représente alkyle, alcényle ou alcynyle,

qui comprend la réaction d'un composé représenté par la formule générale :

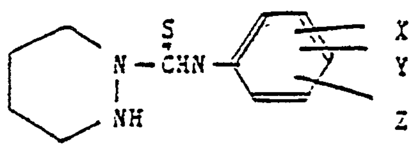

(II)

dans laquelle X, Y et Z sont tels que définis ci-dessus, avec le composé de formule (III) :

$COCl_2$ (III)

en présence ou en l'absence d'une base.

2. Procédé selon la revendication 1, dans lequel Z représente

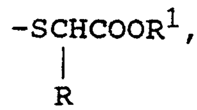

où R et $R^1$ sont tels que définis à la revendication 1.

3. Procédé selon la revendication 1, dans lequel Z représente $-SCH_2COOR^1$, où $R^1$ est tel que défini à la revendication 1.

4. Procédé selon la revendication 1, dans lequel Z représente

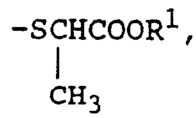

où $R^1$ est tel que défini à la revendication 1.

5. Procédé selon la revendication 1, qui produit un composé représenté par la formule suivante :

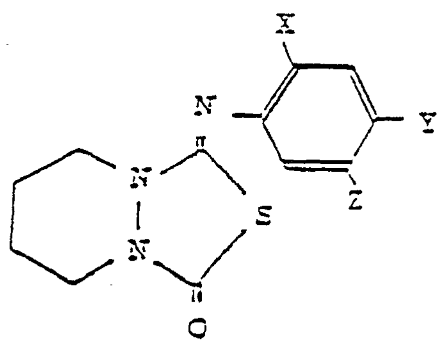

dans laquelle X, Y et Z sont tels que définis à la revendication 1.

6. Procédé selon la revendication 5, dans lequel Z représente

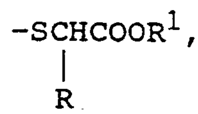

où R et $R^1$ sont tels que définis à la revendication 1.

7. Procédé selon la revendication 6, dans lequel R représente hydrogène ou méthyle.

8. Procédé selon l'une des revendications 1 à 7, dans lequel $R^1$ représente alkyle.

9. Procédé selon l'une des revendications 1 à 8, dans lequel X représente hydrogène ou halogène et Y représente halogène.

10. Procédé selon l'une des revendications 1 à 8, dans lequel X représente hydrogène ou fluor, et Y représente chlore.

11. Composition herbicide comprenant une quantité efficace pour détruire les mauvaises herbes d'un composé de la formule (I), tel que défini à la revendication 1, et un support.

**Patentansprüche**
**Patentansprüche für folgende Vertragsstaaten : BE, CH, DE, FR, GB, IT, LI, NL**

1. 9-Phenylimino-8-thia-1,6-diazabicyclo[4.3.0]nonan-7-on-Derivat mit der Formel:

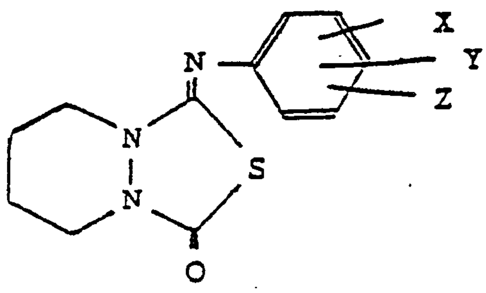

(I)

wobei jedes von X und Y für Wasserstoff oder Halogen steht und Z für

$$-S\underset{\displaystyle R}{\underset{|}{C}}HCOOR^1 ,$$

wobei R Wasserstoff oder Alkyl ist und $R^1$ Alkyl, Cycloalkyl oder Alkoxyalkyl bedeutet, oder für -COOQ steht, wobei Q Alkyl bedeutet, oder Y und Z zusammen

$$-O-\overset{R^2}{\overset{|}{C}}H-\overset{O}{\overset{\|}{C}}-\overset{R^3}{\overset{|}{N}}-\text{bilden,}$$

das an den Phenylring gebunden ist, wobei $R^2$ für Wasserstoff oder Alkyl steht und $R^3$ für Alkyl, Alkenyl oder Alkinyl steht.

2. Verbindung gemäß Anspruch 1, wobei Z für

$$-S\underset{\displaystyle R}{\underset{|}{C}}H-COOR^1$$

steht, wobei R und $R^1$ wie in Anspruch 1 definiert sind.

3. Verbindung gemäß Anspruch 1, wobei Z für $-SCH_2COOR^1$ steht, wobei $R^1$ wie in Anspruch 1 definiert ist.

4. Verbindung gemäß Anspruch 1 wobei Z für

$$-S\underset{\displaystyle CH_3}{\underset{|}{C}}HCOOR^1$$

steht, wobei $R^1$ wie in Anspruch 1 definiert ist.

5. Verbindung gemäß Anspruch 1 mit der Formel

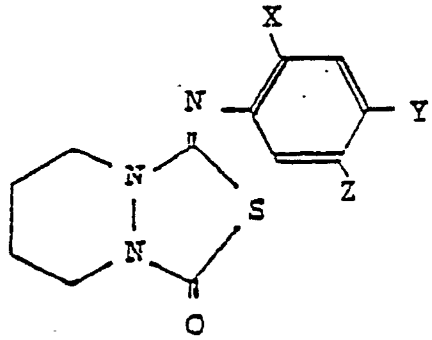

wobei X, Y und Z wie in Anspruch 1 definiert sind.

6. Verbindung gemäß Anspruch 5, wobei Z für

$$-\underset{\displaystyle R}{\underset{|}{S C}}HCOOR^1$$

steht, wobei R und $R^1$ wie in Anspruch 1 definiert sind.

7. Verbindung gemäß Anspruch 6, wobei R für Wasserstoff oder Methyl steht.

8. Verbindung gemäß einem der Ansprüche 1 bis 7, wobei $R^1$ für Alkyl steht.

9. Verbindung gemäß einem der Ansprüch 1 bis 8, wobei X für Wasserstoff oder Halogen steht und Y für Halogen steht.

10. Verbindung gemäß einem der Ansprüche 1 bis 8, wobei X für Wasserstoff oder Fluor steht und Y für Chlor steht.

11. Herbizide Zusammensetzung, umfassend eine herbizid wirksame Menge einer Verbindung der Formel I, wie sie in Anspruch 1 definiert ist, und einen Träger.

**Patentansprüche für folgenden Vertragsstaat : ES**

1. Verfahren zur Herstellung eines 9-Phenylimino-8-thia-1,6-diazabicyclo[4.3.0]nonan-7-on-derivat mit der Formel

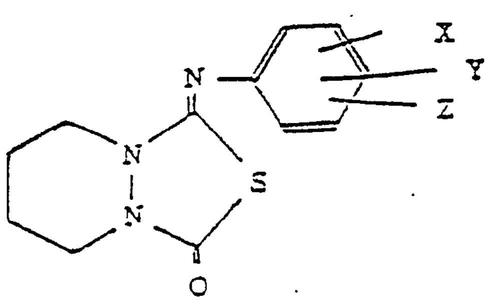

(I)

wobei jedes von X und Y für Wasserstoff oder Halogen steht und Z für

$$-S\underset{\substack{|\\R}}{C}HCOOR^1\ ,$$

wobei R Wasserstoff oder Alkyl ist und $R^1$ Alkyl, Cycloalkyl oder Alkoxyalkyl bedeutet, oder für -COOQ steht, wobei Q Alkyl bedeutet, oder Y und Z zusammen

$$-O-\overset{\substack{R^2\\|}}{C}H-\overset{\substack{O\\\|}}{C}-\overset{\substack{R^3\\|}}{N}-\text{bilden,}$$

das an den Phenylring gebunden ist, wobei $R^2$ für Wasserstoff oder Alkyl steht und $R^3$ für Alkyl, Alkenyl oder Alkinyl steht, umfassend die Umsetzung einer Verbindung der allgemeinen Formel:

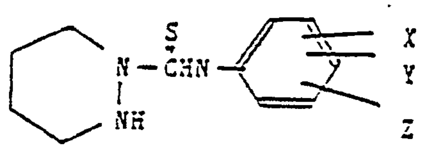

(II)

wobei X, Y und Z die oben angegebene Bedeutung haben, mit der Verbindung der Formel (III)

$COCl_2$ (III)

in Anwesenheit oder Abwesenheit einer Base.

2. Verfahren gemäß Anspruch 1, wobei Z für

$$-S\underset{\substack{|\\R}}{C}HCOOR^1$$

steht, wobei R und $R^1$ wie in Anspruch 1 definiert sind.

3. Verfahren gemäß Anspruch 1, wobei Z für $-SCH_2COOR^1$ steht, wobei $R^1$ wie in Anspruch 1 definiert ist.

4. Verfahren gemäß Anspruch 1, wobei Z für

$$-S\underset{\substack{|\\CH_3}}{C}HCOOR^1$$

steht, wobei $R^1$ wie in Anspruch 1 definiert ist.

5. Verfahren gemäß Anspruch 1, wobei man eine Verbindung der folgenden Formel erhält:

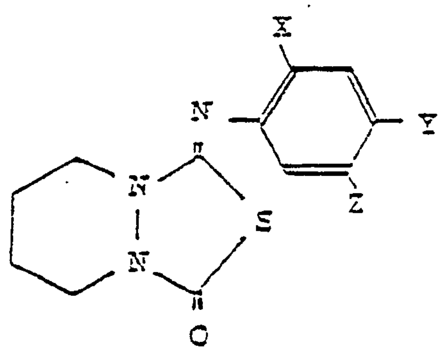

wobei X, Y und Z wie in Anspruch 1 definiert sind.

6. Verfahren gemäß Anspruch 5, wobei Z für

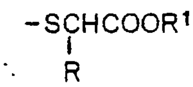

steht, wobei R und $R^1$ wie in Anspruch 1 definiert sind.

7. Verfahren gemäß Anspruch 6, wobei R für Wasserstoff oder Methyl steht.

8. Verfahren gemäß einem der Ansprüche 1 bis 7, wobei $R^1$ für Alkyl steht.

9. Verfahren gemäß einem der Ansprüche 1 bis 8, wobei X für Wasserstoff oder Halogen steht und Y für Halogen steht.

10. Verfahren gemäß einem der Ansprüche 1 bis 8, wobei X für Wasserstoff oder Fluor steht und Y für Chlor steht.

11. Herbizide Zusammensetzung, umfassend eine herbizid wirksame Menge einer Verbindung der Formel I, wie sie in Anspruch 1 definiert ist, und einen Träger.

FIGURE I

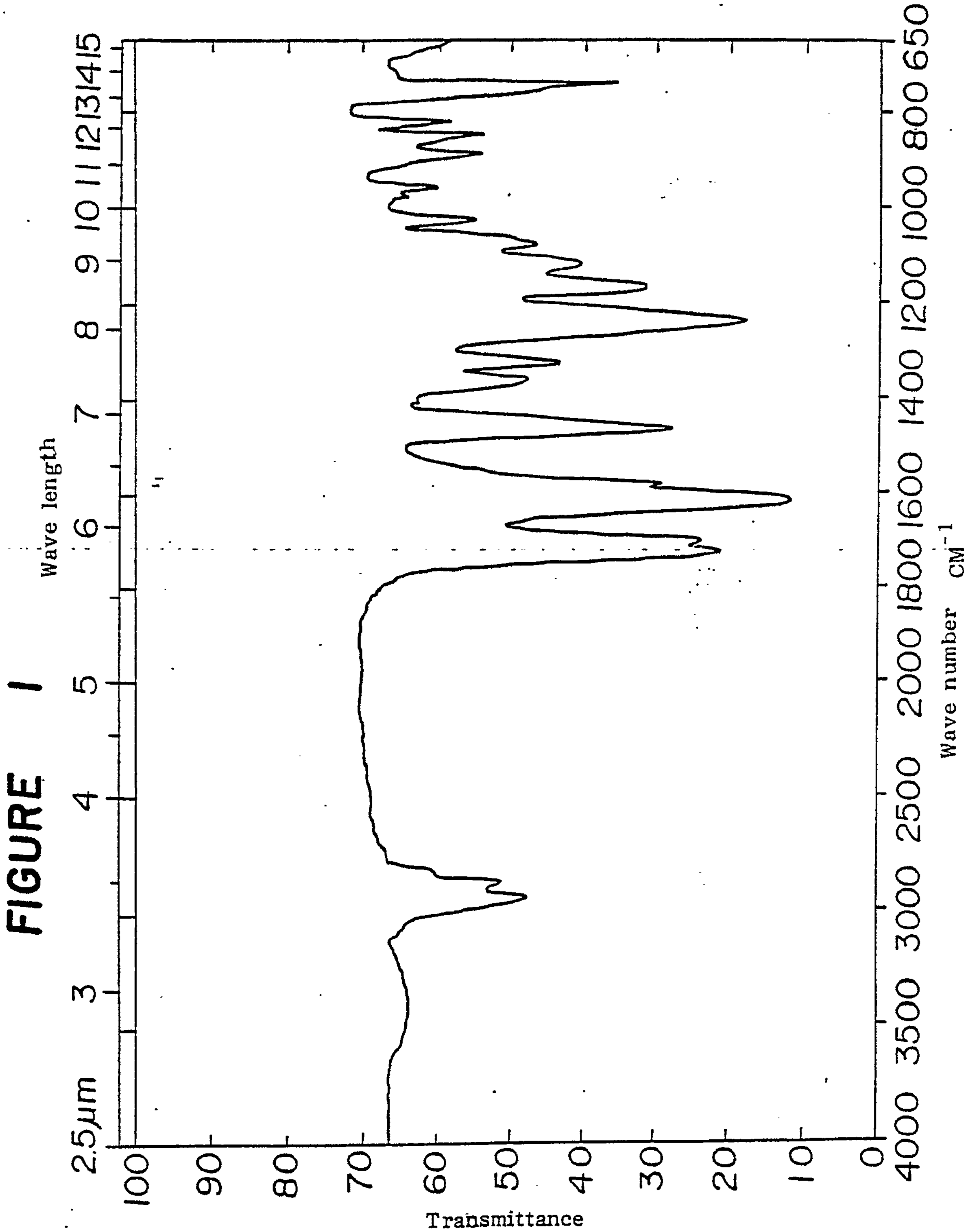

Wave length
2.5μm 3 4 5 6 7 8 9 10 11 12 13 14 15
Wave number CM⁻¹
4000 3500 3000 2500 2000 1800 1600 1400 1200 1000 800 650
Transmittance
100 90 80 70 60 50 40 30 20 10 0